# EUROPEAN PATENT SPECIFICATION

(11) **EP 0 264 906 B1**
(45) Date of publication and mention of the grant of the patent: **08.09.1993**
(21) Application number: 87115336.7
(22) Date of filing: 20.10.1987
(51) Int. Cl.: A61K 7/16, A61K 7/26

(54) **Oral cleaning preparation**
Oralisches Reinigungsmittel
Préparation pour le nettoyage oral

(30) Priority: 21.10.1986 US 921954
(43) Date of publication of application: 27.04.1988
(73) Proprietor: Smith Collins Pharmaceutical, Inc., Riverton Wyoming 82501 (US)
(72) Inventor: Ladanyi, Peter A., Fort Collins Colorado 80524 (US)
(74) Representative: Schwabe - Sandmair - Marx

(56) References cited:
- EP-A- 0 025 649
- US-A- 4 145 412
- US-A- 4 376 115

## Description

The present invention relates to compositions useful for oral hygiene, and in particular to low foaming, soapless, toothpastes and tooth polishing compositions which are formulated to contain various natural extracts.

Various oral hygiene products have been developed for daily use in the care of teeth, gums, and the oral cavity. Such products include flossing materials, gum massage instruments, and, probably most common, tooth cleansing preparations available in the form of tooth pastes, powders, and polishers.

One such composition for use in the oral cavity is described in my U.S. Patent No. 4,145,412. In particular, the invention therein described relates to a toothpaste preparation using a special extract of Sanguinaria canadensis Linne (botanical name) (family Papavaraceae) as a portion of said preparation. Said extract is described to be supplemented with zinc chloride. As there related, Sanguinaria canadensis, commonly known as Bloodroot, Redroot, Puccoon, Teterwort, etc., is a perennial herb native to North America. This herb also has utility as a stimulant expectorant. Additionally, U.S. Patent No. 209,331 describes the use of Bloodroot, zinc chloride, and kerosine oil for open sores. U.S. Patent No. 433,257 describes the use of Bloodroot in a composition for the treatment of piles, while U.S. Patent No. 2,344,830 describes its use with zinc chloride and stibnite (antimony trisulfide) to fix and outline diseased tissue for excising by surgery.

U.S. Patent No. 4,335,110 and European Patent Specification 0 025 649 describes pharmaceutical compositions of Sanguinaria canadensis, Galangal (dried rootstock of Alpina Officinarium), zinc chloride, and deionized water. Such compositions can be used in the treatment of periodontal diseases.

Aloe vera Linne (Aloe barbadensis Miller; family Libisceae) is a succulent perennial herb. The plant and its juices have been used for various medicinal purposes during the course of pre-history as well as recorded history. It has been used as a natural folk remedy medicine for various untoward conditions such as burns, ulcerated conditions, wounds, eczema, stomach ulcers, poison ivy, insect bites and gum diseases.

In view of the various beneficial effects exhibited by such natural compounds, it is an object of the present invention to employ selected natural compounds in a combination which will provide an effective oral hygiene product for daily use in the care of teeth, gums and tissue within the oral cavity.

The subject of the present invention is a composition for use in oral hygiene, said composition comprising Aloe vera gel in an amount of from 0.1 weight-% to 50 weight % of the composition, an extract of Sanguinaria canadensis in an amount of from 0.1 weight-% to 20 weight-% of the composition stabilized by zinc chloride or another suitable salt and a carrier. The carrier can be a compatible liquid, a tooth polishing composition, or it can contain various compounds and compositions associated with tooth cleansing preparations and included, for example, for taste purposes (e.g. sweeteners), for flow properties (e.g. consistency regulators), or for other particular purposes (e.g. stannous fluoride). In this manner an effective tooth cleansing composition is provided which additionally provides breath freshening and cares for gums and tissue within the oral cavity.

The preferred embodiment here described is a toothpaste formulation comprising the following ingredients as shown in Table I.

**Table I**

| Raw Materials | Amount | By Weight |
|---|---|---|
| 1. Sanguinaria Extract in alcohol | 100 g | 2.0 % |
| 2. Zinc chloride | 150 g | 3.0 % |
| 3. Glycerol | 750 g | 15.0 % |
| 4. Sorbitol | 500 g | 10.0 % |
| 5. Polysorbate 80 (Tween 80) | 100 g | 2.0 % |
| 6. Saccharin sodium | 7.5g | 0.15% |
| 7. Tragacanth | 105 g | 2.1 % |
| 8. Dicalcium phosphate (anhydrous) | 300 g | 6.0 % |
| 9. Dicalcium phosphate 2H₂O | 1,850 g | 37.0 % |
| 10. Peppermint oil | 25 g | 0.5 % |
| 11. Menthol | 5 g | 0.1 % |
| 12. Stannous fluoride | 20 g | 0.4 % |
| 13. Water, Deionized | 87.5g | 11.75% |
| 14. Aloe vera gel | 1,000.0g | 10.00% |

The Aloe vera gel is naturally occurring in the Aloe vera plant.

The Sanguinaria canadensis extract is produced by treating a finely cut or ground root of the Sanguinaria plant with an organic solvent such as ethanol. The root is thoroughly stirred with an excess of the solvent, and is maintained in the solvent for at least five days at a temperature of about 25 °C. After such preparation, the solution is filtered and the extract is thereby produced without further treatment. The extract is employed in amounts (by weight) of from 0.1 % to 20% of the final toothpaste composition, and preferably from 1.0% to 10%. Zinc chloride is dissolved in deionized water to form a solution which is filtered and added to the Sanguinaria extract and stirred until completely mixed. The amount of zinc chloride employed can be from 0.1% to 30% by weight of the final toothpaste composition and preferably from 0.1% to 10%. The zinc chloride functions to stabilize the extract.

The resulting stabilized extract is added to the glycerol at a temperature of about 65 °C. and constant stirring for the remainder of all additive additions is begun. The Sorbitol, Polysorbate 80 and saccharin sodium are added, followed by the addition of the tragacanth in small portions to avoid lumping. The anhydrous dicalcium phosphate is added in small portions, followed by portion addition of the dicalcium phosphate dihydrate. Some portions of the deionized water of the toothpaste composition must be added along with the dicalcium phosphate to maintain a good consistency for thorough mixing. Menthol dissolved in peppermint oil is then added, and the remaining water with the containing the stannous fluoride mixed therewith is added. Finally, the Aloe vera is added, and stirring is continued until the desired consistency of the composition is attained. The Aloe vera gel is employed in amounts by weight of the composition of from 0.1% to 50%, and preferably from 5% to 20%.

The composition of the present invention in the form of a toothpaste provides for an easily available oral application of the natural ingredients. This application may be accomplished by the user. Additionally, the composition of the present invention, when in the form of a tooth polishing compound, can advantageously be used for the application of the natural ingredients to the oral cavity by trained oral hygienists.

In the toothpaste preparation Aspartamate or other compatible sweetening agents can be substituted for saccharin; carrageenen or other natural or compatible synthetic gums can be substituted for tragancanth; and sodium fluoride or other suitable fluoride compounds can be substituted for stannous fluoride without altering the toothpaste integrity. Coloring agents can also be added to this preparation without altering the toothpaste quality.

The toothpaste is used in the normal manner, one or more times a day. The low foaming toothpaste is an excellent cleaning agent and breath freshener. Use for about two weeks, on a regular basis, provide improved tissue condition of the oral cavity. A two-week application promotes normal tissue and tends to reduce the bleeding associated with vigorous brushing. Other effects are well cleansed teeth and a refreshed breath.

## Claims (Claims for the following Contracting State(s): AT, BE, CH, DE, FR, GB, IT, LI, LU, NL, SE)

1. A composition for use in oral hygiene comprising:
a. a carrier;
b. an extract of Sanguinaria canadensis in an amount of from 0.1 wt% to 20 wt% of the composition, stabilized by zinc chloride or another suitable salt; and
c. Aloe vera gel in an amount of from 0.1 wt% to 50 wt% of the composition.

2. A composition as claimed in Claim 1 wherein production of the extract of Sanguinaria canadensis comprises maintaining a finely cut or ground root thereof in a solvent for at least five days at 25 °C., filtering the resulting extract, and adding a solution of zinc chloride to stabilize said extract.

3. A composition as claimed in Claim 2 wherein the solvent is ethanol.

4. A composition as claimed in Claims 1, 2 or 3 wherein the extract of Sanguinaria canadensis is present in an amount of from 1 wt% to 10 wt%.

5. A composition as claimed in any of the Claims 1 to 4 wherein the Aloe vera gel is present in an amount of from 5 wt% to 20 wt%.

6. A composition as claimed in any of the Claims 1 to 5 wherein the carrier is a toothpaste base.

7. A composition as claimed in any of the Claims 1 to 5 wherein the carrier is a toothpolishing composition.

8. A composition as claimed in any of the Claims 1 to 5 wherein the carrier is a compatible liquid.

9. A method of producing a composition for use in oral hygiene according to claim 1, said method comprising:
a) producing an extract of Sanguinaria canadensis, said production comprising maintaining a finely cut or ground root thereof in ethanol for at least five days at about 25 °C., filtering the resulting extract, and adding a solution of zinc chloride to stabilize said extract; and
b) mixing said extract of Sanguinaria canadensis with Aloe vera gel and a carrier.

## Claims (Claims for the following Contracting State(s): ES, GR)

1. A process for preparing a composition for use in oral hygiene comprising:
a. a carrier;
b. an extract of Sanguinaria canadensis in an amount of from 0.1 wt% to 20 wt% of the composition, stabilized by zinc chloride or another suitable salt; and
c. Aloe vera gel in an amount of from 0.1 wt% to 50 wt% of the composition.

2. A process as claimed in Claim 1 wherein production of the extract of Sanguinaria canadensis comprises maintaining a finely cut or ground root thereof in a solvent for at least five days at 25 °C., filtering the resulting extract, and adding a solution of zinc chloride to stabilize said extract.

3. A process as claimed in Claim 2 wherein the solvent is ethanol.

4. A process as claimed in Claims 1, 2 or 3 wherein the extract of Sanguinaria canadensis is present in an amount of from 1 wt% to 10 wt%.

5. A process as claimed in any of the Claims 1 to 4 wherein the Aloe vera gel is present in an amount of from 5 wt% to 20 wt%.

6. A process as claimed in any of the Claims 1 to 5 wherein the carrier is a toothpaste base.

7. A process as claimed in any of the Claims 1 to 5 wherein the carrier is a toothpolishing composition.

8. A process as claimed in any of the Claims 1 to 5 wherein the carrier is a compatible liquid.

9. A process of producing a composition for use in oral hygiene according to claim 1, said method comprising:
a) producing an extract of Sanguinaria canadensis, said production comprising maintaining a finely cut or ground root thereof in ethanol for at least five days at about 25 °C., filtering the resulting extract, and adding a solution of zinc chloride to stabilize said extract; and
b) mixing said extract of Sanguinaria canadensis with Aloe vera gel and a carrier.

## Patentansprüche (Patentansprüche für folgende(n) Vertragsstaat(en): AT, BE, CH, DE, FR, GB, IT, LI, LU, NL, SE)

1. Zubereitung zur Verwendung bei der Mundhygiene, die umfaßt
(a) einen Träger;
(b) einen Extrakt von Sanguinaria canadensis in einer Menge von 0,1 Gew.-% bis 20 Gew.-% der Zubereitung, stabilisiert mit Zinkchlorid oder einem anderen geeigneten Salz; und
(c) Aloe vera-Gel in einer Menge von 0,1 Gew.-% bis 50 Gew.-% der Zubereitung.

2. Zubereitung nach Anspruch 1, worin die Herstellung des Extrakts von Sanguinaria canadensis das Aufbewahren einer feingeschnittenen oder gemahlenen Wurzel der Pflanze in einem Lösungsmittel für die Zeit von wenigstens fünf Tagen bei 25 °C, Filtrieren des resultierenden Extraktes und Zusetzen einer Zinkchlorid-Lösung zur Stabilisierung des Extrakts umfaßt.

3. Zubereitung nach Anspruch 2, worin das Lösungsmittel Ethanol ist.

4. Zubereitung nach Ansprüchen 1, 2 oder 3, worin der Extrakt von Sanguinaria canadensis in einer Menge von 1 Gew.-% bis 10 Gew.-% zugegen ist.

5. Zubereitung nach einem der Ansprüche 1 bis 4, worin das Aloe vera-Gel in einer Menge von 5 Gew.-% bis 20 Gew.-% zugegen ist.

6. Zubereitung nach einem der Ansprüche 1 bis 5, worin der Träger ein Zahnpasta-Grundmaterial ist.

7. Zubereitung nach einem der Ansprüche 1 bis 5, worin der Träger eine Zubereitung zum Polieren der Zähne ist.

8. Zubereitung nach einem der Ansprüche 1 bis 5, worin der Träger eine kompatible Flüssigkeit ist.

9. Verfahren zur Herstellung einer Zubereitung zur Verwendung bei der Mundhygiene gemäß Anspruch 1, wobei das Verfahren die Schritte umfaßt, daß man
(a) einen Extrakt von Sanguinaria canadensis herstellt, wobei das Herstellungsverfahren das Aufbewahren einer feingeschnittenen oder gemahlenen Wurzel der Pflanze in Ethanol für die Zeit von wenigstens fünf Tagen bei 25 °C, Filtrieren des resultierenden Extraktes und Zusetzen einer Zinkchlorid-Lösung zur Stabilisierung des Extrakts umfaßt; und
(b) den Extrakt von Sanguinaria canadensis mit Aloe vera-Gel und einem Träger mischt.

## Patentansprüche (Patentansprüche für folgende(n) Vertragsstaat(en): ES, GR)

1. Verfahren zur Herstellung einer Zubereitung zur Verwendung bei der Mundhygiene, die umfaßt
(a) einen Träger;
(b) einen Extrakt von Sanguinaria canadensis in einer Menge von 0,1 Gew.-% bis 20 Gew.-% der Zubereitung, stabilisiert mit Zinkchlorid oder einem anderen geeigneten Salz; und
(c) Aloe vera-Gel in einer Menge von 0,1 Gew.-% bis 50 Gew.-% der Zubereitung.

2. Verfahren nach Anspruch 1, worin die Herstellung des Extrakts von Sanguinaria canadensis das Aufbewahren einer feingeschnittenen oder gemahlenen Wurzel der Pflanze in einem Lösungsmittel für die Zeit von wenigstens fünf Tagen bei 25 °C, Filtrieren des resultierenden Extraktes und Zusetzen einer Zinkchlorid-Lösung zur Stabilisierung des Extrakts umfaßt.

3. Verfahren nach Anspruch 2, worin das Lösungsmittel Ethanol ist.

4. Verfahren nach Ansprüchen 1, 2 oder 3, worin der Extraht von Sanguinaria canadensis in einer Menge von 1 Gew.-% bis 10 Gew.-% zugegen ist.

5. Verfahren nach einem der Ansprüche 1 bis 4, worin das Aloe vera-Gel in einer Menge von 5 Gew.-% bis 20 Gew.-% zugegen ist.

6. Verfahren nach einem der Ansprüche 1 bis 5, worin der Träger ein Zahnpasta-Grundmaterial ist.

7. Verfahren nach einem der Ansprüche 1 bis 5, worin der Träger eine Zubereitung zum Polieren der Zähne ist.

8. Verfahren nach einem der Ansprüche 1 bis 5, worin der Träger eine kompatible Flüssigkeit ist.

9. Verfahren zur Herstellung einer Zubereitung zur Verwendung bei der Mundhygiene gemäß Anspruch 1, wobei das Verfahren die Schritte umfaßt, daß man
(a) einen Extrakt von Sanguinaria canadensis herstellt, wobei das Herstellungsverfahren das Aufbewahren einer feingeschnittenen oder gemahlenen Wurzel der Pflanze in Ethanol für die Zeit von wenigstens fünf Tagen bei 25 °C, Filtrieren des resultierenden Extraktes und Zusetzen einer Zinkchlorid-Lösung zur Stabilisierung des Extrakts umfaßt; und
(b) den Extrakt von Sanguinaria canadensis mit Aloe vera-Gel und einem Träger mischt.

## Revendications (Revendications pour l'(les) Etat(s) contractant(s) suivant(s): AT, BE, CH, DE, FR, GB, IT, LI, LU, NL, SE)

1. Composition destinée à l'usage pour l'hygiène buccale, caractérisée en ce qu'elle comprend :
a. un support ou véhicule;
b. un extrait de Sanguinaria canadensis en une proportion de 0,1% en poids à 20% en poids par rapport à la composition, stabilisé par du chlorure de zinc ou tout autre sel convenable, et
c. de l'Aloe vera en gel en une proportion de 0,1% en poids à 50% en poids par rapport à la composition.

2. Composition suivant la revendication 1, caractérisée en ce que la production de l'extrait de Sanguinaria canadensis comprend le maintien d'une racine finement hachée ou broyée de Sanguinaria canadensis dans un solvant pendant au moins cinq jours à 25°C, la filtration de l'extrait ainsi obtenu et l'addition d'une solution de chlorure de zinc en vue de stabiliser cet extrait.

3. Composition suivant la revendication 2, caractérisée en ce que le solvant est l'éthanol.

4. Composition suivant les revendications 1, 2 ou 3, caractérisée en ce que l'extrait de Sanguinaria canadensis y est présent en une proportion de 1% en poids à 10% en poids.

5. Composition suivant l'une quelconque des revendications 1 à 4, caractérisée en ce que l'Aloa vera en gel y est présent en une proportion de 5% en poids à 20% en poids.

6. Composition suivant l'une quelconque des revendications 1 à 5, caractérisée en ce que le véhicule ou support est une base pour pâte dentifrice.

7. Composition suivant l'une quelconque des revendications 1 à 5, caractérisée en ce que le véhicule ou support est une composition de polissage des dents.

8. Composition suivant l'une quelconque des revendications 1 à 5, caractérisée en ce que le support ou véhicule est un liquide compatible.

9. Procédé de production d'une composition destinée à l'usage pour l'hygiène buccale suivant la revendication 1, caractérisé en ce que
a) on produit un extrait de Sanguinaria canadensis, cette production comprenant le maintien d'une racine finement hachée ou broyée de Sanguinaria canadensis dans l'éthanol pendant au moins cinq jours à environ 25°C, la filtration de l'extrait ainsi obtenu et l'addition d'une solution de chlorure de zinc pour stabiliser l'extrait en question, et
b) on mélange cet extrait de Sanguinaria canadensis à de l'Aloe vera en gel et un véhicule ou support.

## Revendications (Revendications pour l'(les) Etat(s) contractant(s) suivant(s): ES, GR)

1. Procédé de préparation d'une composition destinée à l'usage pour l'hygiène buccale, caractérisé en ce qu'il comprend :
a. un support ou véhicule;
b. un extrait de Sanguinaria canadensis en une proportion de 0,1% en poids à 20% en poids par rapport à la composition, stabilisé par du chlorure de zinc ou tout autre sel convenable, et
c. de l'Aloe vera en gel en une proportion de 0,1% en poids à 50% en poids par rapport à la composition.

2. Procédé suivant la revendication 1, caractérisé en ce que la production de l'extrait de Sanguinaria canadensis comprend le maintien d'une racine finement hachée ou broyée de Sanguinaria canadensis dans un solvant pendant au moins cinq jours à 25°C, la filtration de l'extrait ainsi obtenu et l'addition d'une solution de chlorure de zinc en vue de stabiliser cet extrait.

3. Procédé suivant la revendication 2, caractérisé en ce que le solvant est l'éthanol.

4. Procédé suivant les revendications 1, 2 ou 3, caractérisé en ce que l'extrait de Sanguinaria canadensis y est présent en une proportion de 1% en poids à 10% en poids.

5. Procédé suivant l'une quelconque des revendications 1 à 4, caractérisé en ce que l'Aloa vera en gel y est présent en une proportion de 5% en poids à 20% en poids.

6. Procédé suivant l'une quelconque des revendications 1 à 5, caractérisé en ce que le véhicule ou support est une base pour pâte dentifrice.

7. Procédé suivant l'une quelconque des revendications 1 à 5, caractérisé en ce que le véhicule ou support est une composition de polissage des dents.

8. Procédé suivant l'une quelconque des revendications 1 à 5, caractérisé en ce que le support ou véhicule est un liquide compatible.

9. Procédé de production d'une composition destinée à l'usage pour l'hygiène buccale suivant la revendication 1, caractérisé en ce que
a) on produit un extrait de Sanguinaria canadensis, cette production comprenant le maintien d'une racine finement hachée ou broyée de Sanguinaria canadensis dans l'éthanol pendant au moins cinq jours à environ 25°C, la filtration de l'extrait ainsi obtenu et l'addition d'une solution de chlorure de zinc pour stabiliser l'extrait en question, et
b) on mélange cet extrait de Sanguinaria canadensis à de l'Aloe vera en gel et un véhicule ou support.
